# EUROPEAN PATENT SPECIFICATION

(11) **EP 4 477 150 B1**
(45) Date of publication and mention of the grant of the patent: **18.03.2026**
(21) Application number: 22933741.5
(22) Date of filing: 19.04.2022
(51) Int. Cl.: A61B 6/00, A61B 6/03, G16H 50/20

(54) **ARTIFICIAL INTELLIGENCE-BASED HEAD AND NECK LANDMARK DETECTION METHOD AND DEVICE**
AUF KÜNSTLICHER INTELLIGENZ BASIERENDES KOPF- UND HALS-LANDMARKENDETEKTIONSVERFAHREN UND VORRICHTUNG
PROCÉDÉ ET DISPOSITIF DE DÉTECTION DE POINTS DE REPÈRE DE TÊTE ET DE COU BASÉS SUR L'INTELLIGENCE ARTIFICIELLE

(30) Priority: 24.03.2022 KR 20220036712
(43) Date of publication of application: 18.12.2024
(73) Proprietor: 3D ONS, INC., Seoul 06023 (KR)
(72) Inventor: HONG, Nae Young, Seoul 06023 (KR); PANG, Jin, Seoul 06023 (KR)
(74) Representative: Walaski, Jan Filip
(86) International application number: PCT/KR2022/005622
(87) International publication number: WO 2023/182568

(56) References cited:
- KR-A- 20200 023 703
- KR-A- 20210 071 188
- KR-A- 20210 071 188
- KR-B1- 101 636 876
- KR-B1- 101 952 887
- KR-B1- 102 224 597
- CHENG ZHANGPEI ET AL: "U-Net cascaded with dilated convolution for medical image registration", 2019 CHINESE AUTOMATION CONGRESS (CAC), IEEE, 22 November 2019 (2019-11-22), pages 3647 - 3651, XP033711816, DOI: 10.1109/CAC48633.2019.8996569

## Description

### [Technical Field]

The present invention relates to a method and apparatus for detecting a landmark of the head and neck based on artificial intelligence, and more particularly, to a method and apparatus for detecting a landmark of the head and neck based on artificial intelligence in which a convolutional neural network (CNN) landmark detection model is used to promptly detect an anatomical landmark from a computerized tomography (CT) image of the head and neck.

### [Background Art]

Conventionally, doctors directly perform manual work to select the positions of anatomically significant landmarks from a three-dimensional computerized tomography (CT) image of the head and neck of a patient. Since there are limitations in checking and analyzing a plurality of landmarks by visual inspection, and the positions of landmarks may be different for each person, there is a problem in terms of accuracy.

In order to address such a problem, there have been attempts to introduce artificial intelligence technology in place of humans. However, since letting a model learn large-capacity three-dimensional CT images as learning data requires a large operation quantity, there are problems in that the speed is low and the cost is high. Also, there is a problem in that learning is difficult because images output from a plurality of different CT apparatuses have different densities.

Korean Patent Publication No. 10-2021-0071188 (Prediction apparatus for predicting anatomical landmarks and a prediction method thereof) is one prior art document, but the prior art document only discloses a technology for predicting the positions of anatomical landmarks based on landmark and patient characteristics from a patient's cephalometric image.

### [Disclosure]

### [Technical Problem]

The present invention is directed to providing a method and apparatus for detecting a landmark of the head and neck based on artificial intelligence capable of promptly and accurately detecting an anatomically significant landmark through artificial intelligence technology from large-capacity three-dimensional computerized tomography (CT) images of the head and neck.

### [Technical Solution]

The present invention provides a method for detecting a landmark of the head and neck based on artificial intelligence according to claim 1.

The present invention also provides an apparatus for detecting a landmark of the head and neck based on artificial intelligence according to claim 3.

### [Advantageous Effects]

According to the present invention, by primarily detecting a landmark through a landmark detection model by converting a three-dimensional computerized tomography (CT) image into a low-resolution CT image and secondarily detecting a landmark in relation to a detected specific area, landmarks of the head and neck can be promptly and accurately detected.

Also, by letting various apparatuses learn captured CT images of the head and neck, landmark detection is universally possible without being dependent on a specific apparatus.

### [Description of Drawings]

FIG. 1 is a flowchart for describing a method for detecting a landmark of the head and neck based on artificial intelligence according to an embodiment of the present invention.
FIG. 2 is a block diagram for describing an apparatus for detecting a landmark of the head and neck based on artificial intelligence according to an embodiment of the present invention.
FIG. 3 is a conceptual diagram for describing the method for detecting a landmark of the head and neck based on artificial intelligence according to an embodiment of the present invention.
FIG. 4 is a conceptual diagram for describing a landmark detection model according to an embodiment of the present invention.
FIG. 5 is a conceptual diagram for describing a method in which the landmark detection model performs learning according to an embodiment of the present invention.
FIG. 6 is a conceptual diagram for describing a method of learning CT images output from different apparatuses according to an embodiment of the present invention.
FIG. 7 is an exemplary view showing the results of landmark detection according to an embodiment of the present invention.

### [Modes of the Invention]

The specific structural or functional description is merely illustrative for the purpose of describing embodiments according to the concept of the present invention with respect to embodiments according to the concept of the present invention disclosed herein. Embodiments according to the concept of the present invention may be implemented in various forms, and may not be construed as limited to the embodiments set forth herein.

Since embodiments according to the concept of the present invention may be changed in various ways and have various forms, the embodiments are illustrated in the drawings and will be described in detail herein. However, it is not intended to limit the embodiments according to the concept of the present invention to specific disclosed forms, and the present invention includes all changes, equivalents, or substitutes included in the spirit and technical scope of the present invention.

Terms used herein are only used to describe specific embodiments and are not intended to limit the present invention. A singular expression includes a plural expression unless the context clearly indicates otherwise. In the present specification, terms such as "include" or "have" should be understood as indicating the presence of features, numbers, steps, operations, elements, parts, or combinations thereof and not excluding the possibility of the presence or addition of one or more other features, numbers, steps, operations, elements, parts, or combinations thereof in advance.

Hereinafter, embodiments of the present invention will be described in detail with reference to the accompanying drawings.

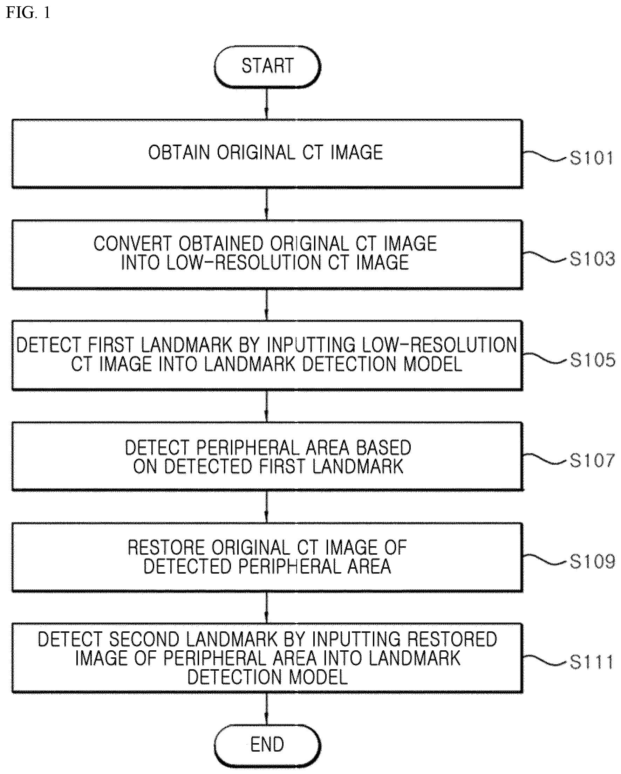

FIG. 1 is a flowchart for describing a method for detecting a landmark of the head and neck based on artificial intelligence according to an embodiment of the present invention.

Referring to FIG. 1, an original computerized tomography (CT) image obtainer obtains an original CT image of the head and neck of a patient (S101). The original CT image is a three-dimensional CT image of the head and neck of a patient that is obtained by cone beam CT or general medical CT.

An image converter converts the obtained original CT image into a low-resolution CT image (S103). Since original CT images have a large capacity, there is a problem in that a large operation quantity places a burden on hardware in letting a convolutional neural network (CNN)-based landmark detection model perform learning or detect landmarks. In order to address such a problem, in the present invention, preprocessing is performed to convert the obtained original CT image into the low-resolution CT image.

A landmark detector detects a first landmark by inputting the low-resolution CT image into a landmark detection model (S105). The landmark detection model is a CNN-based application model and may primarily output coordinates of the first landmark when the low-resolution CT image is input. The landmark detection model consists of down-sampling for outputting only a representative feature as a small image from the input image, up-sampling for re-extracting only the output representative feature at a size corresponding to the original image size, and a skip layer for extracting detailed position information and the feature together.

A peripheral area detector detects a peripheral area based on the coordinates of the detected first landmark of the head and neck (S107). The peripheral area detector may detect a rectangular parallelepiped peripheral area with the coordinates of the first landmark as a central point. Here, the shape of the peripheral area is not limited to a rectangular parallelepiped shape, and peripheral areas of various other shapes may be detected according to embodiments. Then, an image restorer restores an original CT image of the detected peripheral area (S109). The image restorer restores a high-resolution original CT image only for the detected peripheral area.

The landmark detector detects a second landmark by inputting the restored image of the peripheral area into the landmark detection model (S111). That is, by secondarily detecting a landmark by restoring the original CT image only for the detected peripheral area, a landmark can be detected from a high-resolution three-dimensional CT image.

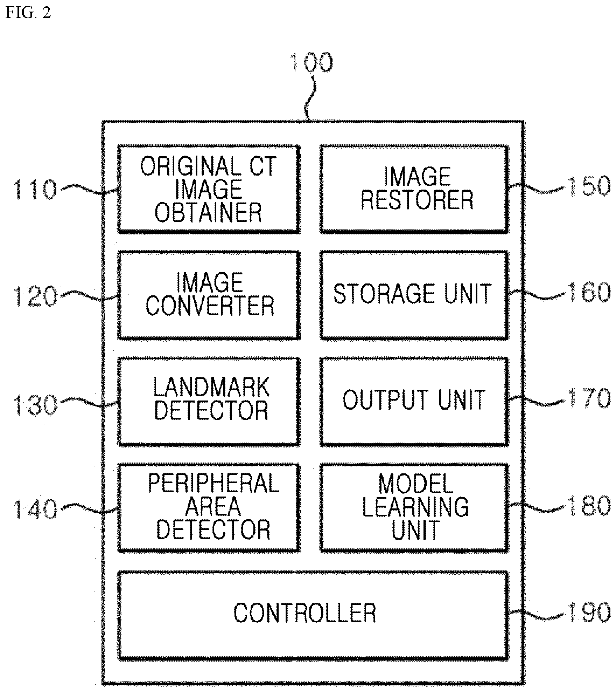

FIG. 2 is a block diagram for describing an apparatus for detecting a landmark of the head and neck based on artificial intelligence according to an embodiment of the present invention.

Referring to FIG. 2, a landmark detection device 100 consists of an original CT image obtainer 110, an image converter 120, a landmark detector 130, a peripheral area detector 140, an image restorer 150, a storage unit 160, an output unit 170, a model learning unit 180, and a controller 190.

The original CT image obtainer 110 obtains an original CT image of the head and neck of a patient. The image converter 120 converts the obtained original CT image into a low-resolution CT image.

The landmark detector 130 detects a first landmark by inputting the low-resolution CT image into a landmark detection model and detects a second landmark by inputting a restored image of a peripheral area into the landmark detection model.

The peripheral area detector 140 detects a peripheral area based on the detected first landmark of the head and neck. The peripheral area has a cubic or rectangular parallelepiped shape, but the shape of the peripheral area is not limited thereto. The size of the peripheral area may be adjusted according to an input of an administrator.

The image restorer 150 restores an original CT image for only the detected peripheral area. The image restorer 150 may enlarge only the detected peripheral area to restore the original CT image.

The storage unit 160 stores the coordinates of the detected first landmark and second landmark and stores the original CT image, the low-resolution CT image, the detected peripheral area, and the landmark detection model.

The output unit 170 may display and output a plurality of second landmarks on the original CT image. The model learning unit 180 may input the original CT image into the landmark detection model to let the landmark detection model perform learning in advance. The model learning unit 180 may input CT images input from a plurality of apparatuses into the landmark detection model to let the landmark detection model perform learning in advance.

The controller 190 may perform control by having software embedded therein for controlling each component of the landmark detection device.

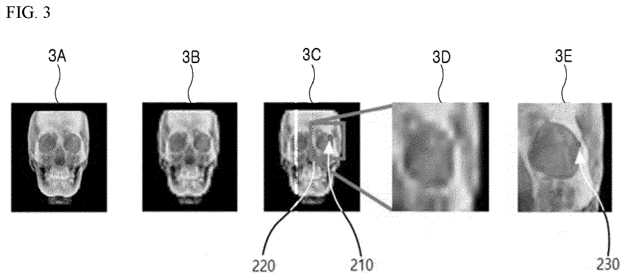

FIG. 3 is a conceptual diagram for describing the method for detecting a landmark of the head and neck based on artificial intelligence according to an embodiment of the present invention. Referring to FIG. 3, an original CT image (3A) of the head and neck of a patient is converted into a low-resolution CT image (3B), and the low-resolution CT image is input into a landmark detection model to detect a first landmark 210 (3C). A peripheral area 220 having a cubic or rectangular parallelepiped shape of a predetermined size is detected with the coordinates of the detected first landmark as a central point (3C). The peripheral area 220 part is enlarged (3D), an original CT image is restored only for the peripheral area 220 part, and the original CT image is secondarily input into the landmark detection model to detect a second landmark. That is, according to the present invention, by primarily detecting a landmark through a landmark detection model by converting a three-dimensional CT image into a low-resolution CT image and secondarily detecting a landmark in relation to a detected specific area, landmarks of the head and neck can be promptly and accurately detected.

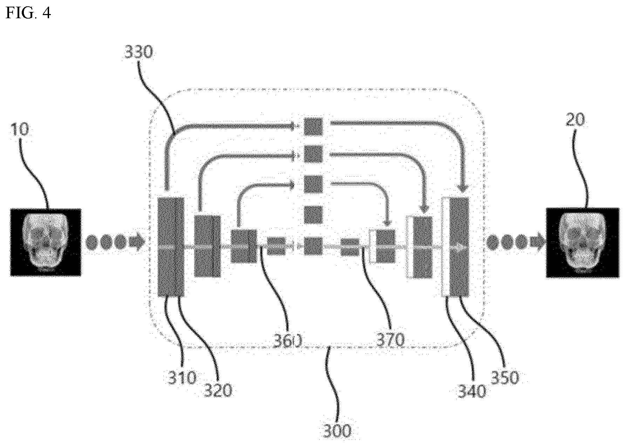

FIG. 4 is a conceptual diagram for describing a landmark detection model according to an embodiment of the present invention.

Referring to FIG. 4, a landmark detection model 300 consists of down-sampling (360) for outputting only a representative feature as a small image from an input image, up-sampling (370) for re-extracting only the output representative feature at a size corresponding to the original image size, and a skip layer (330) for extracting detailed position information and the feature together. Through the process from down-sampling to up-sampling, the overall contour and the feature of the input image may be extracted. The landmark detection model may detect a landmark by combining the overall contour and the detailed feature and position information and may output an image displaying the detected landmark.

The down-sampling (360) consists of a convolution layer 310 configured to extract a feature from an image and output the feature as an image of the same size and a maxpooling layer 320 configured to extract only a representative feature from the image and output the representative feature as a smaller image, and the down-sampling (360) may repeatedly go through the convolution layer 310 and the maxpooling layer 320 to output the representative feature as a small image.

The up-sampling (370) may output a feature as a large image through a nearest neighbor 340, which is configured to increase the size of the small image output by the down-sampling, and a convolution layer 350, and the up-sampling may be repeatedly performed until the size of the output image becomes equal to the size of the input image. The up-sampling may be repeated the same number of times as the down-sampling.

The skip layer separately extracts images that are about to pass through the convolution layer in each step of the down-sampling process, lets the images pass through a separate convolution layer, and then outputs resulting features as an image. The output image is taken to the up-sampling operation and merged with an image of the same size. Here, as a merging method, values between voxels of the same index value on the images are added, and since the skip layer does not use the maxpooling layer or the nearest neighbor when extracting a feature, the skip layer may deliver the images while maintaining position information of the voxels.

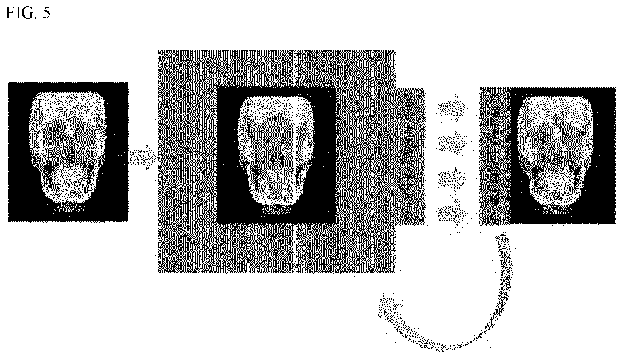

FIG. 5 is a conceptual diagram for describing a method in which the landmark detection model performs learning according to an embodiment of the present invention. Referring to FIG. 5, the landmark detection model may learn position relations of related landmarks. The landmark detection model is designed to detect a single landmark from a single image and may be re-designed to simultaneously detect several landmarks according to the embodiment. The content relating to re-design will be described with reference to FIG. 5. In the re-design, the number of landmarks of a learning image may be increased to several landmarks, and the output of the landmark detection model may also be increased to correspond to the number of landmarks. When simultaneously learned landmarks are present in this way, the landmark detection model may learn the landmarks in consideration of the position relations between the landmarks. That is, since the position relations between the landmarks are formed simultaneously as a plurality of pieces of landmark information are input as feedback, the landmark detection model may learn the position relations between the landmarks. For example, when learning several landmarks, the landmark detection model learns the landmarks in consideration of the position relations between the landmarks, such as the chin being positioned lower than the eyes. When the landmark detection model learns the position relations between the landmarks in this way, even when there are differences in characteristics of three-dimensional CT images output from different apparatuses, an error such as learning that the position of a chin tip landmark is higher than a pupil landmark may be prevented. Also, by using the position relations between landmarks as additional information beyond simply finding feature points from characteristics of an image itself, a landmark can be more accurately detected.

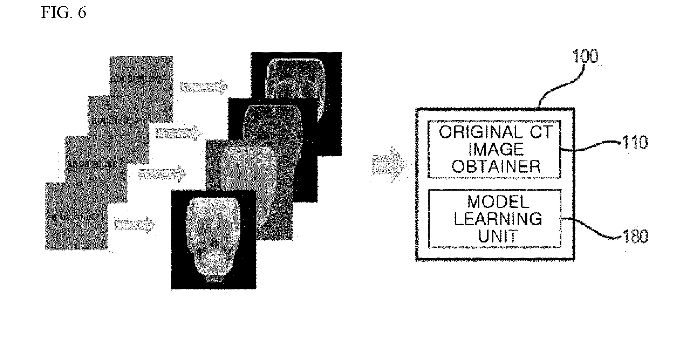

FIG. 6 is a conceptual diagram for describing a method of learning CT images output from different apparatuses according to an embodiment of the present invention. Referring to FIG. 6, CT images output from different apparatuses may be provided as learning data, and the landmark detection model may learn and infer the learning data regardless of differences between the apparatuses. Three-dimensional CT images output from different apparatuses have a problem in that they have different density value characteristics, and the landmark detection model may learn the three-dimensional CT images having various density values as learning data.

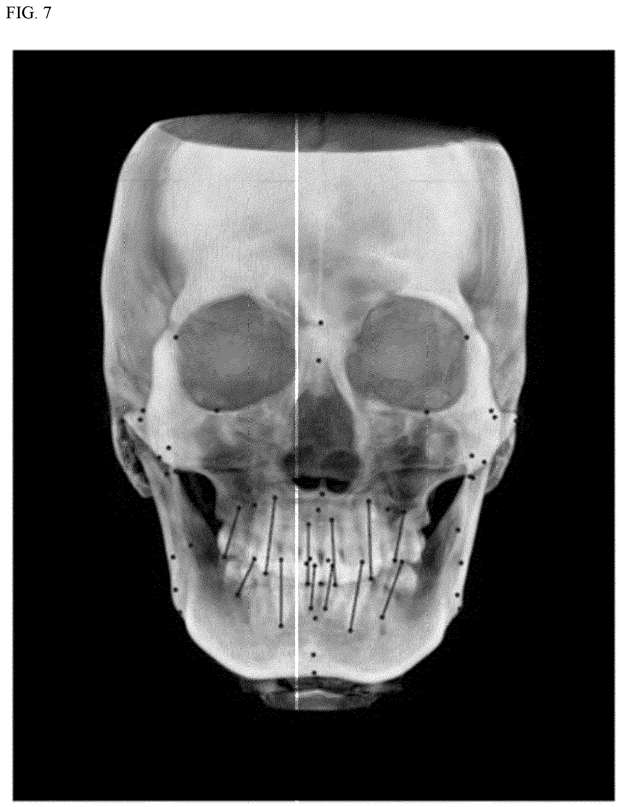

FIG. 7 is an exemplary view showing the results of landmark detection according to an embodiment of the present invention. Referring to FIG. 7, a plurality of anatomically significant landmarks detected from a three-dimensional original CT image are illustrated.

### [Description of reference numerals]

| | | | |
|---|---|---|---|
| 100: | landmark detection device | 110: | original CT image obtainer |
| 120: | image converter | 130: | landmark detector |
| 140: | peripheral area detector | 150: | image restorer |
| 160: | storage unit | 170: | output unit |
| 180: | model learning unit | 190: | controller |

## Claims

1. A method for detecting a landmark of the head and neck based on artificial intelligence, the method comprising:
an operation in which an original computerized tomography (CT) image obtainer obtains an original CT image of the head and neck of a patient (S101);
an operation in which an image converter converts the obtained original CT image into a low-resolution CT image (S103);
an operation in which a landmark detector detects a first landmark by inputting the low-resolution CT image into a landmark detection model (5105);
an operation in which a peripheral area detector detects a peripheral area based on the detected first landmark of the head and neck (S107), wherein the peripheral area is a region of interest within the CT image centred at the first landmark,;
an operation in which an image restorer restores an original CT image of the detected peripheral area (S109); and
an operation in which the landmark detector detects a second landmark by inputting the restored image of the peripheral area into the landmark detection model (S111),
wherein the landmark detection model outputs coordinates of the first landmark in response to the input low-resolution CT image and outputs coordinates of the second landmark in response to the input image of the peripheral area of the first landmark,
wherein the landmark detection model consists of down-sampling for outputting only a representative feature as a small image from the input image, up-sampling for re-extracting only the output representative feature at a size corresponding to the original image size, and a skip layer for extracting detailed position information and the feature together.

2. The method of claim 1, wherein the down-sampling consists of a convolution layer configured to extract a feature from an image and output the feature as an image of the same size and a maxpooling layer configured to extract only a representative feature from the image and output the representative feature as a smaller image, and the down-sampling repeatedly goes through the convolution layer and the maxpooling layer to output the representative feature as a small image.

3. An apparatus for detecting a landmark of the head and neck based on artificial intelligence (100), the apparatus comprising:
an original computerized tomography (CT) image obtainer (110) configured to obtain an original CT image of the head and neck of a patient;
an image converter (120) configured to convert the obtained original CT image into a low-resolution CT image;
a landmark detector (130) configured to detect a first landmark by inputting the low-resolution CT image into a landmark detection model and detect a second landmark by inputting a restored image of a peripheral area into the landmark detection model; and
a peripheral area detector (140) configured to detect a peripheral area based on the detected first landmark of the head and neck, wherein the peripheral area is a region of interest within the CT image centred at the first landmark,
wherein the landmark detection model outputs coordinates of the first landmark in response to the input low-resolution CT image and outputs coordinates of the second landmark in response to the input image of the peripheral area of the first landmark,
wherein the landmark detection model consists of down-sampling for outputting only a representative feature as a small image from the input image, up-sampling for re-extracting only the output representative feature at a size corresponding to the original image size, and a skip layer for extracting detailed position information and the feature together.

## Patentansprüche

1. Verfahren zum Erkennen eines Orientierungspunkts des Kopfes und des Halses basierend auf künstlicher Intelligenz, wobei das Verfahren Folgendes umfasst:
eine Operation, bei der ein Original-Computertomographie(CT)-Bilderlanger ein Original-CT-Bild des Kopfes und Halses eines Patienten erlangt (S101);
eine Operation, bei der ein Bildkonverter das erlangte Original-CT-Bild in ein CT-Bild mit niedriger Auflösung konvertiert (S103);
eine Operation, bei der ein Orientierungspunktdetektor einen ersten Orientierungspunkt durch Eingeben des CT-Bilds mit niedriger Auflösung in ein Orientierungspunktdetektionsmodell (S105) erkennt;
eine Operation, bei der ein Umfangsbereichsdetektor einen Umfangsbereich basierend auf dem erkannten ersten Orientierungspunkt des Kopfes und des Halses erkennt (S107), wobei der Umfangsbereich ein Bereich von Interesse innerhalb des CT-Bilds ist, der auf den ersten Orientierungspunkt zentriert ist;
eine Operation, bei der ein Bildwiederhersteller ein Original-CT-Bild des erkannten Umfangsbereichs wiederherstellt (S109); und
eine Operation, bei der der Orientierungspunktdetektor einen zweiten Orientierungspunkt erkennt, indem er das wiederhergestellte Bild des Umfangsbereichs in das Orientierungspunktdetektionsmodell eingibt (S111),
wobei das Orientierungspunktdetektionsmodell Koordinaten des ersten Orientierungspunkts als Reaktion auf das eingegebene CT-Bild mit niedriger Auflösung ausgibt und Koordinaten des zweiten Orientierungspunkts als Reaktion auf das eingegebene Bild des Umfangsbereichs des ersten Orientierungspunkts ausgibt,
wobei das Orientierungspunktdetektionsmodell aus einem Downsampling zum Ausgeben nur eines repräsentativen Merkmals als kleines Bild aus dem Eingabebild, einem Upsampling zum erneuten Extrahieren nur des ausgegebenen repräsentativen Merkmals in einer Größe, die der ursprünglichen Bildgröße entspricht, und einer Skip-Schicht zum Extrahieren detaillierter Positionsinformationen und des Merkmals zusammen besteht.

2. Verfahren nach Anspruch 1, wobei das Downsampling aus einer Convolution-Schicht, die dazu konfiguriert ist, ein Merkmal aus einem Bild zu extrahieren und das Merkmal als ein Bild der gleichen Größe auszugeben, und einer Maxpooling-Schicht besteht, die dazu konfiguriert ist, nur ein repräsentatives Merkmal aus dem Bild zu extrahieren und das repräsentative Merkmal als ein kleineres Bild auszugeben, und das Downsampling wiederholt die Convolution-Schicht und die Maxpooling-Schicht durchläuft, um das repräsentative Merkmal als ein kleines Bild auszugeben.

3. Vorrichtung zum Erkennen eines Orientierungspunkts des Kopfes und des Halses basierend auf künstlicher Intelligenz (100), wobei die Vorrichtung Folgendes umfasst:
einen Original-Computertomographie(CT)-Bilderlanger (110), der zum Erlangen eines Original-CT-Bildes des Kopfes und Halses eines Patienten konfiguriert ist;
einen Bildkonverter (120), der zum Konvertieren des erlangten Original-CT-Bildes in ein CT-Bild mit niedriger Auflösung konfiguriert ist;
einen Orientierungspunktdetektor (130), der zum Erkennen eines ersten Orientierungspunkts durch Eingeben des CT-Bilds mit niedriger Auflösung in ein Orientierungspunktdetektionsmodell und zum Erkennen eines zweiten Orientierungspunkts durch Eingeben eines wiederhergestellten Bildes eines Umfangsbereichs in das Orientierungspunktdetektionsmodell konfiguriert ist; und
einen Umfangsbereichsdetektor (140), der zum Erkennen eines Umfangsbereich basierend auf dem erkannten ersten Orientierungspunkt des Kopfes und des Halses konfiguriert ist, wobei der Umfangsbereich ein Bereich von Interesse innerhalb des CT-Bilds ist, der auf den ersten Orientierungspunkt zentriert ist;
wobei das Orientierungspunktdetektionsmodell Koordinaten des ersten Orientierungspunkts als Reaktion auf das eingegebene CT-Bild mit niedriger Auflösung ausgibt und Koordinaten des zweiten Orientierungspunkts als Reaktion auf das eingegebene Bild des Umfangsbereichs des ersten Orientierungspunkts ausgibt,
wobei das Orientierungspunktdetektionsmodell aus einem Downsampling zum Ausgeben nur eines repräsentativen Merkmals als kleines Bild aus dem Eingabebild, einem Upsampling zum erneuten Extrahieren nur des ausgegebenen repräsentativen Merkmals in einer Größe, die der ursprünglichen Bildgröße entspricht, und einer Skip-Schicht zum Extrahieren detaillierter Positionsinformationen und des Merkmals zusammen besteht.

## Revendications

1. Procédé permettant la détection d'un point de repère de la tête et du cou basé sur l'intelligence artificielle, le procédé comprenant :
une opération dans laquelle un dispositif d'obtention d'image de tomodensitométrie par ordinateur (TDM) originale obtient une image TDM originale de la tête et du cou d'un patient (S101) ;
une opération dans laquelle un convertisseur d'image convertit l'image TDM originale obtenue en une image TDM à basse résolution (S103) ;
une opération dans laquelle un détecteur de point de repère détecte un premier point de repère en entrant l'image TDM à basse résolution dans un modèle de détection de point de repère (S105) ;
une opération dans laquelle un détecteur de zone périphérique détecte une zone périphérique sur la base du premier point de repère détecté de la tête et du cou (S107), ladite zone périphérique étant une région d'intérêt à l'intérieur de l'image TDM centrée au niveau du premier point de repère ;
une opération dans laquelle un restaurateur d'image restaure une image TDM originale de la zone périphérique détectée (S109) ; et
une opération dans laquelle le détecteur de point de repère détecte un second point de repère en entrant l'image restaurée de la zone périphérique dans le modèle de détection de point de repère (S111),
ledit modèle de détection de point de repère délivrant en sortie des coordonnées du premier point de repère en réponse à l'image TDM à basse résolution d'entrée et délivrant en sortie des coordonnées du second point de repère en réponse à l'image d'entrée de la zone périphérique du premier point de repère,
ledit modèle de détection de point de repère étant constitué d'un sous-échantillonnage pour délivrer en sortie uniquement une caractéristique représentative sous forme d'une petite image à partir de l'image d'entrée, un sur-échantillonnage pour extraire de nouveau uniquement la caractéristique représentative de sortie à une taille correspondant à la taille de l'image originale, et une couche de saut pour extraire ensemble des informations de position détaillées et la caractéristique.

2. Procédé de la revendication 1, ledit sous-échantillonnage étant constitué d'une couche de convolution configurée pour extraire une caractéristique d'une image et délivrer en sortie la caractéristique sous la forme d'une image de la même taille et une couche de regroupement maximal configurée pour extraire uniquement une caractéristique représentative de l'image et délivrer en sortie la caractéristique représentative sous la forme d'une image plus petite, et ledit sous-échantillonnage passant de manière répétitive par la couche de convolution et la couche de regroupement maximal pour délivrer en sortie la caractéristique représentative sous la forme d'une petite image.

3. Appareil destiné à la détection d'un point de repère de la tête et du cou basé sur l'intelligence artificielle (100), l'appareil comprenant :
un dispositif d'obtention d'image de tomodensitométrie par ordinateur (TDM) originale (110) configuré pour obtenir une image TDM originale de la tête et du cou d'un patient ;
un convertisseur d'image (120) configuré pour convertir l'image TDM originale obtenue en une image TDM à basse résolution ;
un détecteur de point de repère (130) configuré pour détecter un premier point de repère en entrant l'image TDM à basse résolution dans un modèle de détection de point de repère et détecter un second point de repère en entrant une image restaurée d'une zone périphérique dans le modèle de détection de point de repère ; et
un détecteur de zone périphérique (140) configuré pour détecter une zone périphérique sur la base du premier point de repère détecté de la tête et du cou, ladite zone périphérique étant une région d'intérêt à l'intérieur de l'image TDM centrée au niveau du premier point de repère,
ledit modèle de détection de point de repère délivrant en sortie des coordonnées du premier point de repère en réponse à l'image TDM à basse résolution d'entrée et délivrant en sortie des coordonnées du second point de repère en réponse à l'image d'entrée de la zone périphérique du premier point de repère,
ledit modèle de détection de point de repère étant constitué d'un sous-échantillonnage pour délivrer en sortie uniquement une caractéristique représentative sous forme d'une petite image à partir de l'image d'entrée, un sur-échantillonnage pour extraire de nouveau uniquement la caractéristique représentative de sortie à une taille correspondant à la taille de l'image originale, et une couche de saut pour extraire ensemble des informations de position détaillées et la caractéristique.
